# EUROPÄISCHE PATENTANMELDUNG

(11) **EP 1 157 982 A2**
(43) Veröffentlichungstag der Anmeldung: **28.11.2001**
(21) Anmeldenummer: 01111729.8
(22) Anmeldetag: 15.05.2001
(51) Int. Cl.: C07C 69/54, C07C 67/58

(54) **Verfahren zum Reinigen von durch Cracken von Polymethacrylaten gewonnenen Methacrylsäureestern**

(30) Priorität: 26.05.2000 DE 10025945
(71) Anmelder: Röhm GmbH & Co. KG, 64293 Darmstadt (DE)
(72) Erfinder: Carl, Joachim, Dr., 64342 Seeheim-Jugenheim (DE); Wittkowski, Andrea, 64823 Gross-Umstadt (DE)

(57) **Zusammenfassung**

Aus dem Rohprodukt der PMMA-Depolymerisation läßt sich durch Reinigung mit Säuren (H₂SO₄) oder Basen (NaOH) und anschließende Neutralisation und Destillation ein wieder polymerisierbares MMA gewinnen.

Die Menge an Reinigungsmittel ist gering und liegt bei ca. 5 Gew.-%, bezogen auf das Rohprodukt.

## Beschreibung

Die Erfindung betrifft ein Verfahren entsprechend dem Oberbegriff des Anspruchs 1. Die Depolymerisation von Polymethylmethacrylat (PMMA) erfolgt durch Einwirkung von Wärme durch Spaltung der Makromoleküle in ihre Monomere Methylmethacrylat (MMA).

### STAND DER TECHNIK

Es ist bekannt, daß zur thermischen Depolymerisation von PMMA eine Reihe von verschiedenen Verfahren und Reaktoren eingesetzt werden können. In der Literatur werden genannt die trockene Destillation (US-PS 2,030,901), der Einsatz von überhitztem Dampf als Wärmeübertragungsmedium (Kauter DE 729 730), das Aufschmelzen auf Schneckenextrudern und das Einspeisen in einen Pyrolysereaktor (DE 31 46 194), die Wirbelschichtpyrolyse (Kaminsky in Brandrup Merkes Michaeli Bittner, Hansa Verlag, München, 2. Auflage; Titel "Wiederverwertung von Kunstsotffen") und die Verwendung von aufgeschmolzenen Metall- oder Metallsalzbädern (US-PS 2,858,255).

DE-OS 197 290 65 (IKV, Aachen) beschreibt die Depolymerisation von PMMA unter Verwendung von Depolymerisationsbeschleunigungen, wie beispielsweise 2,3-Dimethyl-2,3-diphenyl-butan.

DE-OS 21 32 716 (Rohm & Haas Cooperation, Philadelphia) beschreibt ein Verfahren zur Depolymerisation von Polymethylmethacrylat auf einer geschmolzenen Metalloberfläche, beispielsweise Blei, Zinn oder Cadmium bei Temperaturen über 400 °C. Die Oberfläche des geschmolzenen Metalls muß durch mechanische Rühren ständig erneuert werden. Es ist notwendig, den Reaktor mit einer inerten Atmosphäre aus Stickstoff, Argon oder Helium zu beschicken. Man erreicht einen Gehalt des Monomeren Methylmethacrylat im Destillat zwischen 90,6 und 85,7 %.

US-PS 2,030,901 beschreibt ein Verfahren zur trocknen Destillation von PMMA.

DRP 734 956 (Röhm & Haas GmbH) beschreibt ein Verfahren zum Reinigen von Crack-MMA mit konzentrierter Schwefelsäure. Auf 10 Teile rohes Crack-Produkt werden 1 bis 20 Teile konzentrierte Schwefelsäure eingesetzt. Nach einer gewissen Behandlungszeit bei Temperaturen nicht über 50 °C wird eine Menge Wasser hinzugesetzt, so daß sich 2 Schichten bilden, anschließend wird die MMA-haltige Schicht mit Natronlauge neutralisiert. Die Gesamtausbeute an wiederpolymerisierbaren Ester beträgt zwischen 82 und 85 %.

All diesen Verfahren ist gemeinsam, daß die Polymeren auf eine Temperatur über ihre Ceiling-Temperatur erhitzt werden und die Verbindungen sich daher zersetzten. Es entstehen immer Nebenprodukte, die eine aufwendige Reinigung des Monomeren notwendig machen.

### AUFGABE

Durch die hohe Temperatur bedingt, laufen nicht nur die erwünschten Depolymerisationsreaktionen ab, sondern es entstehen in unkontrollierter und unkontrollierbarer Weise aus den üblichen Zusatzverbindungen zu extrudiertem oder gegossenen PMMA noch jede Menge anderer organischer Verbindungen. Je nach dem, welches PMMA eingesetzt wird, befinden sich daher auch noch Zersetzungsprodukte der Regler, der Initiatoren, der Comonomere und sonstiger Verarbeitungshilfstoffe dabei. Man kann sowohl gegossenes PMMA (PMMA-GS) als auch extrudiertes PMMA (PMMA-XT) im Depolymerisationsprozeß einsetzen.

Ferner kann das im Crackprozeß des Standes der Technik eingesetzte PMMA durch weitere Kunststoffe, die nicht PMMA sind, verunreinigt sein, wie beispielsweise Polyethylen, Polypropylen oder auch Polyvinylchlorid.

Das entstehende Crack-MMA riecht daher extrem übel, ist gelb gefärbt und kann nicht in den Polymerisationsprozess eingesetzt werden. Eine beispielhafte Analyse eines durch Pyrolyse gewonnenen MMA's ergibt folgende Zusammensetzung:

| | |
|---|---|
| MMA | 88,54 Gew.-% |
| Summe an Niedrigsiedern | 3,69 Gew.-% |
| Summe an Hochsiedern | 7,77 Gew.-% |
| Anzahl Peaks | 312 |

Diese Messung erfolgte mit Hilfe eines Gaschromatographen und eines Flammenionisationsdetektors. Dieses Pyrolyse-MMA wird in einer 2-stufigen Destillation (1. Stufe: Leichtsieder werden von MMA abgetrennt, 2. Stufe: MMA wird von den Hochsiedern abgetrennt) zu Crack-MMA aufgereinigt.

Reinigungsschema:
PMMA → Pyrolyse-MMA
Pyrolyse-MMA → Crack-MMA

Selbst dieses durch zweimalige Destillation aufgereinigte Crack-MMA riecht extrem übel und kann daher nicht in den üblichen Polymerisationsreaktionen weiter verarbeitet werden.

### LÖSUNG

Es wurde nun gefunden, daß durch die Behandlung des Pyrolyse-MMA's mit verschiedenen Reagenzien, wie beispielsweise Alkalien oder Säuren eine deutliche Geruchsminderung bei Crack-MMA erhalten wird.

Als Säure kommt beispielsweise konzentrierte Schwefelsäure oder Phosphorsäure in Betracht, als Alkalien kann man NaOH (wasserfrei), und/oder und Ammoniak verwenden, ebenso kann KOH oder Ca(OH)₂ verwendet werden.

Das Pyrolyse-PMMA kann auch, gegebenenfalls nach vorheriger Trocknung, mit Na-Metall behandelt werden. Man verwendet hierzu ein Verfahren wie es in der
DE 28 13 200 zur Enthalogenierung von gebrauchten Schmierölen beschrieben wird.

### Begriffsdefinitionen:

- Virgin-MMA:: Unter Virgin-MMA verstehen wir hier ein MMA, das direkt durch die Aceton-cyanhydrinsynthese aus Blausäure und Aceton gewonnen wird und einen Reinheitsstandard darstellt.
- Crack-MMA:: MMA, das durch Pyrolyse von PMMA gewonnen wurde und anschließend mit einem 2-stufigen Destillationsprozeß (1. Stufe - Leichtsieder wurden vom MMA abgetrennt, 2. Stufe - MMA wird von den Hochsiedern abgetrennt) vorgereinigt wurde.
- Pyrolyse-MMA:: MMA, das durch Pyrolyse von MMA gewonnen und nicht weiter gereinigt wurde. Als Pyrolyseverfahren kommen
alle Verfahren des Standes der Technik in Frage.
- NS:: **N**ieder**s**ieder, Verunreinigung die niedriger als MMA bei Normaldurck sieden
- HS:: **H**och**s**ieder, Verunreinigung die höher als MMA bei Normaldruck sieden

### BEISPIELE

### 1. Beispiel zur Reinigung mit Säure

In einer Standard-1l-Rührapparatur (Kolben, Rückflußkühler, Rührer, Tropftrichter, Gaseinleitungsrohr, Kolonne, Heizbad mit Temperaturreglung) werden 500 g Pyrolyse-MMA mit Stabilisator (250 mg Hydrochinonmonomethylether) vorgelegt, das Reagenz zugefügt und unter Lufteinleiten und Rühren auf Siedetemperatur (ca. 92 °C) erhitzt.

Nach der Reaktionszeit (siehe Tabelle) wird der Ansatz neutralisiert (siehe Tabelle) und anschließend filtriert. Das Filtrat wird bei Normaldruck fraktioniert destilliert, wobei die Hauptfraktion bei einer Kopftemperatur von 98,8 - 99,2 °C übergeht.

### 2. Beispiele 3 - 5

In einer 1 I Standard-Rührapparatur wird das Pyrolyse-MMA, Stabilisator und das entpsrechende Deodorierungsreagenz zur Behandlung vorgelegt, unter Lufteinleiten und Rühren wird auf Siedetemperatur des MMA (ca. 92 °C) erwärmt. Nach der jeweiligen Reaktionszeit wird der Ansatz gegebenenfalls neutralisiert und anschließend filtriert. Das Filtrat wird bei Normaldruck fraktioniert destilliert, wobei die Hauptfraktion bei einer Kopftemperatur von 98,8 - 99,2 °C übergeht.

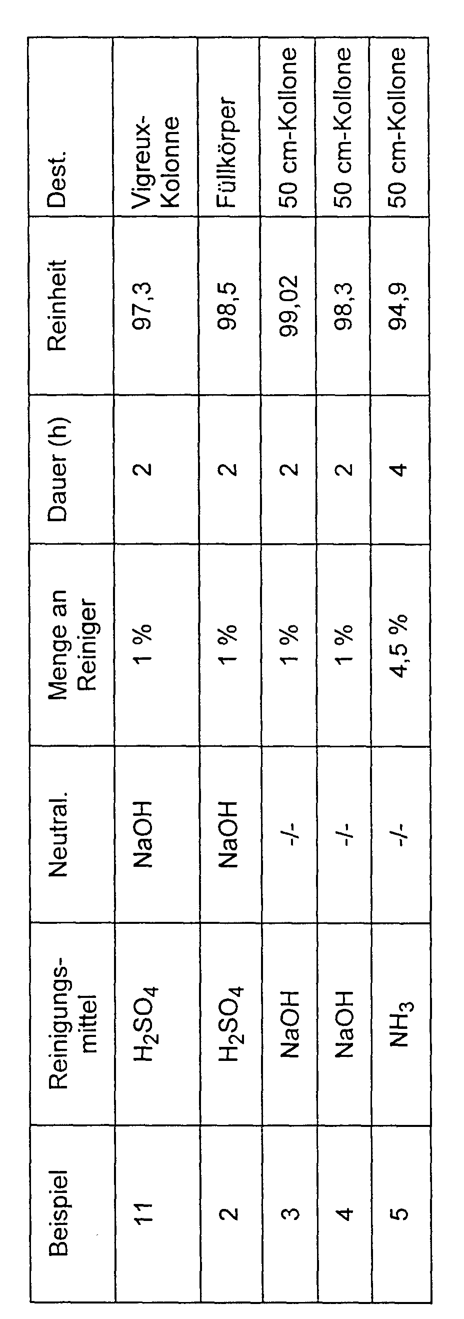

| Qualitative Auswertung der Gaschromatogramme | | | |
|---|---|---|---|
| | Pyrolyse MMA | Virgin MMA | Beispiel 3 |
| Summe der Peaks | 312 | 7 | 27 |
| MMA | 88,5 % | 99,986 | 99,02 |

## Patentansprüche

1. Verfahren zum Reinigen von durch Cracken von Polymethacrylaten gewonnenen Methacrylsäureestern,
**dadurch gekennzeichnet**
**daß** man das nach dem Cracken anfallende Rohprodukt mit konzentrierten Säuren oder Laugen behandelt und anschließend neutralisiert.

2. Verfahren nach Anspruch 1,
**dadurch gekennzeichnet,**
**daß** man als Säure konzentrierte Schwefelsäure verwendet.

3. Verfahren nach Anspruch 2,
**dadurch gekennzeichnet,**
**daß** man die Schwefelsäure, in einer Menge bis zu 5 Gew.-%, bezogen auf das Crack-MMA, einsetzt.

4. Verfahren nach Anspruch 3,
**dadurch gekennzeichnet,**
**daß** man die Schwefelsäure, in einer Menge bis zu 1 Gew.-%, bezogen auf das Crack-MMA einsetzt.

5. Verfahren nach Anspruch 1,
**dadurch gekennzeichnet,**
**daß** man wasserfreies NaOH, KOH oder Ca(OH)₂ einsetzt.

6. Verfahren nach Anspruch 1,
**dadurch gekennzeichnet,**
**daß** man wasserfreies NH₃ einsetzt.

7. Verfahren nach Anspruch 5,
**dadurch gekennzeichnet,**
**daß** man das wasserfreie NaOH in einer Menge bis zu 5 Gew.-%, bezogen auf das Crack-MMA, einsetzt.

8. Verfahren nach Anspruch 7,
**dadurch gekennzeichnet,**
**daß** man das wasserfreie NaOH in einer Menge bis zu 1 Gew.-%, bezogen auf das Crack-MMA, einsetzt.

9. Verfahren nach Anspruch 6,
**dadurch gekennzeichnet,**
**daß** man das wasserfreie NH₃ in einer Menge bis zu 5 %, bezogen auf das Crack-MMA, einsetzt.

10. Verfahren nach Anspruch 9,
**dadurch gekennzeichnet,**
**daß** man das wasserfreie NH₃ in einer Menge bis zu 1 %, bezogen auf das Crack-MMA, einsetzt.
